# EUROPEAN PATENT APPLICATION

(11) **EP 1 457 780 A1**
(43) Date of publication of application: **15.09.2004**
(21) Application number: 02792030.5
(22) Date of filing: 26.12.2002
(51) Int. Cl.: G01N 33/50

(54) **DRUGS FOR AMELIORATING ITCH, ROUGH SKIN OR HYPERSENSITIVE SKIN OR FOR WHITENING VIA INHIBITION OF THE PRODUCTION AND RELEASE OF STEM CELL FACTOR**

(30) Priority: 27.12.2001 JP 2001397571
(71) Applicant: SHISEIDO COMPANY LIMITED, Chuo-ku, Tokyo 104-8010 (JP)
(72) Inventor: ASHIDA, Yutaka c/o SHISEIDO RESEARCH CENTER, Yokohama-shi, Kanagawa 224-8558 (JP); AOKI, Hirofumi c/o SHISEIDO RESEARCH CENTER, Yokohama-shi, Kanagawa 224-8558 (JP); FUJIWARA, Rumiko c/o SHISEIDO RESEARCH CENTER, Yokohama-shi, Kanagawa 224-8558 (JP)
(74) Representative: Santarelli
(86) International application number: PCT/JP2002/013713
(87) International publication number: WO 2003/056331

(57) **Abstract**

The invention provides a screening method for active ingredients which exhibit effects of ameliorating pruritus, rough skin or sensitive skin, or effects of skin whitening, by inhibiting production and/or release of stem cell factor (SCF), as well as medicaments for pruritus, rough skin, sensitive skin and/or skin whitening which contain the active ingredients.

## Description

### Technical Field

The present invention relates to a screening method for active ingredients which exhibit the effects of ameliorating pruritus, rough skin or sensitive skin, or the effect of skin whitening, by inhibiting production and/or release of stem cell factor (hereinafter, "SCF"), as well as to medicaments for pruritus, rough skin, sensitive skin and/or for skin whitening which contain the active ingredients.

### Background Art

Various therapeutic agents, skin external preparations, cosmetics and the like have traditionally been used for ameliorating pruritus, rough skin or sensitive skin or for whitening skin. As active ingredients for conventional medications or cosmetics having effects of ameliorating pruritus, rough skin or sensitive skin, there have been used anti-inflammatory agents, or amino acids, polysaccharides, lipids or the like, as well as various animal or plant extracts with anti-inflammatory effects or high moisture absorbing effects, because of their excellent abilities to prevent skin itching, inflammation or cornified layer moisture loss. As active ingredients for conventional medications or cosmetics exhibiting whitening effects, there have been used L-ascorbic acid, glutathione, kojic acid, cysteine, hydroquinones, placenta extract and the like, because of their excellent abilities to inhibit melanin production and thus prevent skin spots, freckles and the like. However, all of their effects have been less than satisfactory, and the development of superior medicaments has therefore been desired.

It is known that expression of SCF (also referred to as "kit ligand" (KL) or mast cell growth factor (MCF)) is accelerated in skin spot areas, etc., and that ultraviolet irradiation accelerates expression of SCF (L.H. Kligman et al., Photochem. Photobiol. Vol.63, No.2 (1996) pp.123-127). SCF is a protein produced by keratinocytes, fibroblasts, vascular endothelial cells and the like. The effects of SCF include effects of growth of undifferentiated hematopoietic stem cells, promoted differentiation of reproductive cells, promoted growth of mast cells and promoted growth of pigment cells (Bio Science Terminology Library - Cytokines and Growth Factors, Yodosha Publications (1995), ed. by K. Miyazono and K. Sugamura). Two forms of SCF are known, the membrane-bound form (SCF-2) and the secreted form (SCF-1) which is liberated from the membrane after cleavage by proteases. SCF-2 binds to SCF receptors on pigment cells while bound to cornified or other types of cells, thereby activating growth of the pigment cells, whereas SCF-1 is cleaved at its cleavage site and liberated from the cell membrane and then binds to SCF receptors on pigment cells or mast cells, resulting in growth activation of pigment cells or growth activation and degranulation of mast cells (T. Kunisada et al., J. Exp. Med., Vol.187, No.10, (1998) pp.1565-1573). Abnormal production of SCF is linked with abnormal proliferation of pigment cells, which results in accelerated melanin production and is a cause of skin spots, freckles, darkened skin and the like. It is also associated with abnormal proliferation and abnormal degranulation of mast cells, resulting in accelerated release of chemical mediators such as histamine, serotonin and LTB4 (J. Grabbe et al., Arch. Dermatol. Res. (1984) 287:78-84), and constitutes a cause of pruritus, rough skin, sensitive skin and similar conditions.

Thus, it is believed that the ability to effectively inhibit production and release of SCF, which is considered to be the direct cause of pruritus, rough skin, sensitive skin, skin spots, freckles and the like, should lead to completely novel and more effective medicaments for pruritus, rough skin, sensitive skin and/or for skin whitening which do not exist in the prior art. In the prior art, however, no effective screening method is known for substances or galenicals with such inhibiting activity on SCF production and release. T. Kunisada et al. (supra) have examined the action of SCF on mast cells and pigment cells by animal experiments using transgenic mice. However, because such animal experiments result in high costs and take a long time they have not been a suitable means for screening a wide and diverse variety of galenicals and medicaments, and therefore it has been desired to develop a method which allows convenient and economical screening of active ingredients and galenicals having inhibiting activities on SCF production and release.

Surprisingly, the present inventors found that SCF production or release can be promoted by stimulation, in a manner such as drying, of human epidermal keratinocytes and, as a result, succeeded in efficiently screening for active ingredients which can effectively inhibit production and/or release of SCF.

### Disclosure of the Invention

Specifically, according to a first mode of the invention, there is provided a screening method for active ingredients which exhibit effects of ameliorating pruritus, rough skin or sensitive skin, or effects of skin whitening, by inhibiting production and/or release of SCF, the method being characterized by comprising the steps of contacting SCF-expressing cells with test ingredients, assaying the amount of SCF produced and/or released by the cells, and selecting test ingredients which reduce the amount of production and/or release of SCF as the active ingredients, wherein the SCF-expressing cells are subjected to stimulation to promote SCF production and/or release.

According to one aspect of this mode, the stimulation is drying stimulation, ultraviolet irradiation stimulation or chemical stimulation, and is preferably drying stimulation.

According to another aspect of this mode, the screening method comprises contacting the SCF-expressing cells with the test ingredients, subsequently subjecting the cells to stimulation, and then assaying the amount of SCF produced and/or released by the cells and selecting the active ingredients.

According to a second mode of the invention, there are provided skin external preparations which inhibit SCF production and/or release, which comprise one or more ingredients selected from the group consisting of rose extract rose water, camellia sinensis leaf extract, hops extract, hawthorn extract, adzuki bean powder, white birch extract, cinnamon extract, clove extract, arnica extract, peony extract, lime, chlorella extract, Roman chamomile extract, black tea extract, eucalyptus extract, powdered cang zhu extract, powdered bai zhu extract, powdered oolong tea extract, restharrow extract, uncaria gambir extract, grape leaf extract, saposhnikovia root extract, mulberry bark extract, parietaria extract, benzoin extract, stevia extract, cypress extract, calamus extract, soybean extract, Chinese cat's claw extract, soapwort extract, althaea extract, otogiriso (*Hypericum erectum*) extract and artemisia extract.

According to a third mode of the invention, there are provided skin external preparations for pruritus which comprise ingredients which inhibit production and/or release of SCF as pruritus-ameliorating active ingredients.

According to one aspect of this mode, the pruritus-ameliorating active ingredients are one or more selected from the group consisting of hops extract, hawthorn extract, adzuki bean powder, clove extract, Roman chamomile extract, black tea extract, eucalyptus extract, powdered cang zhu extract, powdered bai zhu extract, powdered oolong tea extract, restharrow extract, saposhnikovia root extract, parietaria extract, benzoin extract, stevia extract, calamus extract, Chinese cat's claw extract, soapwort extract, althaea extract and otogiriso (*Hypericum erectum*) extract.

According to a fourth mode of the invention, there are provided skin external preparations for rough skin prevention which comprise ingredients which inhibit production and/or release of SCF as rough skin-preventing active ingredients.

According to one aspect of this mode, the rough skin-preventing active ingredients are one or more selected from the group consisting of hawthorn extract, powdered cang zhu extract, powdered bai zhu extract, restharrow extract, mulberry bark extract, parietaria extract, benzoin extract, stevia extract, cypress extract, calamus extract, Chinese cat's claw extract, soapwort extract and althaea extract.

According to a fifth mode of the invention there are provided skin external preparations for sensitive skin which comprise ingredients which inhibit production and/or release of SCF as sensitive skin-ameliorating active ingredients.

According to one aspect of this mode, the sensitive skin-ameliorating active ingredients are one or more selected from the group consisting of rose extract rose water, camellia sinensis leaf extract, hops extract, hawthorn extract, adzuki bean powder, white birch extract, cinnamon extract, clove extract, chlorella extract, black tea extract, eucalyptus extract, powdered cang zhu extract, powdered bai zhu extract, powdered oolong tea extract, restharrow extract, uncaria gambir extract, grape leaf extract, saposhnikovia root extract, mulberry bark extract, parietaria extract, benzoin extract, stevia extract, cypress extract, calamus extract, soybean extract, Chinese cat's claw extract, soapwort extract, althaea extract, otogiriso (*Hypericum erectum*) extract and artemisia extract.

According to a sixth mode of the invention there are provided skin external preparations for skin whitening which comprise ingredients which inhibit production and/or release of SCF as skin whitening active ingredients.

According to one aspect of this mode, the skin whitening active ingredients are one or more selected from the group consisting of rose extract rose water, restharrow extract, uncaria gambir extract, parietaria extract, benzoin extract, soapwort extract, chlorella extract and eucalyptus extract.

According to a seventh mode of the invention, there is provided a method for ameliorating pruritus, rough skin or sensitive skin or whitening skin, which comprises applying, on to human or mammalian epidermis, an active ingredient which exhibits an effect of ameliorating pruritus, rough skin or sensitive skin, or an effect of skin whitening, by inhibiting production and/or release of SCF.

According to an eighth mode of the invention, there are provided medicaments comprising ingredients which inhibit SCF expressed inside or on the cell membranes of keratinocytes in response to ultraviolet ray stimulation.

According to one aspect of this mode, the ingredients are one or more selected from the group consisting of rose extract rose water, camellia sinensis leaf extract, hops extract, hawthorn extract, adzuki bean powder, white birch extract, cinnamon extract, clove extract, arnica extract, peony extract, lime, chlorella extract, Roman chamomile extract, black tea extract, eucalyptus extract, powdered cang zhu extract, powdered bai zhu extract, powdered oolong tea extract, restharrow extract, uncaria gambir extract, grape leaf extract, saposhnikovia root extract, mulberry bark extract, parietaria extract, benzoin extract, stevia extract, cypress extract, calamus extract, soybean extract, Chinese cat's claw extract, soapwort extract, althaea extract, otogiriso (*Hypericum erectum*) extract and artemisia extract.

According to another aspect of this mode, the ingredients are adzuki bean powder, arnica extract, restharrow extract and benzoin extract.

The extracts of the aforementioned modes were obtained by the methods described below and provided for testing.
[1] Extracts of artemisia, cinnamon, adzuki bean powder, calamus and stevia were obtained by ordinary methods after extraction for several hours in water, as the solvent, with heating from room temperature to 50°C.
[2] Extracts of soapwort, hawthorn, clove, althaea, lime, peony, eucalyptus, benzoin, cypress, oolong tea, mulberry bark, soybean and uncaria gambir were obtained by ordinary methods after soaking and extraction at room temperature for one week in hydrous ethanol (30% ethanol) as the solvent.
[3] Extract of black tea was obtained by an ordinary method after extraction for 5 hours in water or hydrous ethanol (30% ethanol) as the solvent, with heating (60°C).
[4] Extracts of white birch, camellia sinensis leaf, otogiriso, restharrow, arnica, parietaria, chlorella, rose extract rose water, grape leaf, hops and Roman chamomile were obtained by ordinary methods after heated extraction at 60°c for 3 hours, or at 90°C for 2 hours, in 80% or 90% ethanol water as the solvent.

### Brief Description of Drawings

Fig. 1 is a graph showing inhibition of SCF by various galenical extracts on cells subjected to drying stimulation.
Fig. 2 is a graph showing inhibition of SCF by various galenical extracts on cells subjected to drying stimulation.
Fig. 3 is a graph showing acceleration of SCF expression by cells under ultraviolet ray stimulation.
Fig. 4 is a graph showing acceleration of SCF expression by cells under chemical stimulation.
Fig. 5 is a graph showing inhibition of SCF by various galenical extracts on cells subjected to ultraviolet ray stimulation.

### Best Mode for Carrying Out the Invention

The present invention will now be described in detail.

The invention provides a screening method for active ingredients which exhibit effects of ameliorating pruritus, rough skin or sensitive skin, or effects of skin whitening, by inhibiting production and/or release of SCF. The screening method is characterized by contacting SCF-expressing cells with test ingredients, assaying the amount of SCF produced and/or released by the cells, and selecting test ingredients which reduce the amount of production and/or release of SCF as the active ingredients, wherein the SCF-expressing cells are subjected to stimulation to promote SCF production and/or release.

The present inventors have discovered that stimulation such as drying of skin cells including human keratinocytes results in accelerated SCF production and/or release by the cells. Thus, subjecting cells to such stimulation can induce abnormal expression of SCF in such cells, leading to outbreak of pruritus, rough skin, sensitive skin and skin spots, freckles and the like. Utilizing this knowledge, the present inventors accelerated expression of SCF by stimulation to promote production and/or release of SCF while allowing test ingredients which inhibit production and/or release of SCF to act on cells, and have thereby succeeded in efficiently screening for active ingredients which effectively inhibit production and/or release of SCF. Such stimulation includes drying stimulation, as well as other types of stress such as ultraviolet irradiation stimulation, heat stimulation (heating or cooling), chemical stimulation (for example, forskolin or theophyllin), osmotic stimulation and oxidizing stimulation.

The stimulation of cells according to the screening method of the invention may be carried out before, during or after allowing a test ingredient to act on cells. The cells are preferably subjected to the stimulation after allowing the test ingredient to act on the cells.

More specifically, the screening method may be carried out, for example, in the following manner.
(1) SCF-producing cells are cultured in an appropriate culturing solution (for example, at about 25 to 37°C, and preferably at about 37°C, for a period of a few hours to several days, and preferably for about 72 hours).
(2) The test ingredient is diluted to a desired concentration (for example, 0.0001%-0.01%) with appropriate cell culturing solution.
(3) The cell culture solution of (1) is exchanged with the aforementioned diluted test ingredient and incubated (for example, at about 25 to 37°C, and preferably at about 37°C for a period of a few hours to several days, and preferably about 24 hours). Cells with equivalent activity may also optionally be prepared in medium containing no test ingredient as a control.
(4) The cells on which the active ingredient have been allowed to act are subjected to stimulation. When the stimulation is drying stimulation, the supernatant of the cell culture solution is removed and incubation is conducted under drying conditions. Non-stimulated cells may also optionally be prepared as a control.
(5) Medium is added to the stimulated cells and incubation is carried out (for example, at about 25 to 37°C, and preferably at about 37°C for a period of about 30 minutes to 4 hours, and preferably about 2 hours).
(6) The cell supernatant is collected, the SCF concentration is assayed and ingredients which inhibit production and/or release of SCF are selected.

Inhibition of an amount of SCF production and/or release is defined as a reduction of about 25% of SCF production with addition of the test ingredient, as compared to 100% as the value obtained with addition of the medium alone. However, the test ingredient must be added in a concentration range which does not cause notable cytotoxicity (for example, a reduction to about 75% in respiratory activity).

The SCF-producing cells used may be human or other mammalian cells, and for example, they may be keratinocytes, fibroblasts, vascular endothelial cells, etc. derived from a rat, mouse, rabbit or the like. They are preferably human keratinocytes.

As mentioned above, stimulation includes stresses such as drying stimulation, ultraviolet irradiation stimulation, heat stimulation (heating and cooling), chemical stimulation (for example, forskolin and theophyllin), osmotic stimulation, oxidizing stimulation and the like. In the case of drying stimulation, for example, the cells may be subjected to drying stimulation by removal of the medium in an incubator with an atmosphere of about 1 to 5% CO₂, a humidity of about 0 to 100% and a temperature of 25 to 37°C, followed by standing for about 15 minutes to 6 hours, and preferably about 1 hour. In the case of ultraviolet irradiation stimulation, for example, the cells may be stimulated by ultraviolet irradiation at a wavelength of about 290 to 320 nm at 10 to 60 mJ/cm². For heat stimulation, for example, the cells may be stimulated by standing in a CO₂ incubator with an atmosphere of about 1 to 5% CO₂, a humidity of about 0 to 100% and a temperature of 4 to 25°C or 37 to 42°C for an appropriate period of time, such as from about 5 minutes to 6 hours, and preferably about 1 hour.

The screening method of the invention is preferably conducted *in vitro,* but may also be carried out *in vivo.*

### Assay of SCF

According to the invention, the SCF produced and/or released by the SCF-expressing cells is preferably assayed by qualitative or quantitative assay of SCF released by the cells into the cell culture solution or SCF present in the disrupted cells or cell membrane fraction. The SCF to be assayed may be the secreted form (SCF-1) which is liberated from the cell membrane and released into the cell culture solution, or the membrane-bound form (SCF-2) included in the cell membrane fraction. As SCF assay methods, there may be mentioned various methods well known in the technical field such as, for example, immunoassay methods including ELISA assays utilizing enzyme labels, RIA methods utilizing radioactive labels, immunonephelometric methods wherein the amount of antigen is quantitated based on changes in absorbance of the turbidity produced upon reaction of antibody with the antigen, and latex agglutination or hemagglutination methods wherein the amount of antigen is assayed based on the degree of agglutination produced by reaction between antigen and antibody-sensitized latex beads or antibody-sensitized erythrocytes. The immunoassay system may be a competitive or a sandwich assay system. The immunoassay may also be accomplished by electrophoresis, isoelectric focusing or chromatography, such as gel filtration chromatography, ion-exchange chromatography, reversed-phase chromatography, high performance liquid chromatography, or Western blotting. According to the invention, the SCF assay is preferably accomplished by ELISA.

SCF-specific antibodies used for the above-mentioned immunoassay method may be monoclonal antibodies or polyclonal antibodies, but monoclonal antibodies are especially preferred. Methods for production of monoclonal antibodies and polyclonal antibodies are well known to those skilled in the art.

Using the screening method of the invention, the present inventors have found that the following galenicals reduce SCF production by SCF-producing cells:
Rose extract rose water, camellia sinensis leaf extract, hops extract, hawthorn extract, adzuki bean powder, white birch extract, cinnamon extract, clove extract, arnica extract, peony extract, lime, chlorella extract, Roman chamomile extract, black tea extract, eucalyptus extract, powdered cang zhu extract, powdered bai zhu extract, powdered oolong tea extract, restharrow extract, uncaria gambir extract, grape leaf extract, saposhnikovia root extract, mulberry bark extract, parietaria extract, benzoin extract, stevia extract, cypress extract, calamus extract, soybean extract, Chinese cat's claw extract, soapwort extract, althaea extract, otogiriso (*Hypericum erectum*) extract and artemisia extract. Detailed descriptions of these galenicals may be found in Nippon Hanyou Keshouhin Genryoshu, 4th Edition (Yakuji Nippo).

Extracts of the aforementioned galenicals are obtained from crude plant materials by ordinary methods with no restrictions on the extraction methods, and for example in the case of clove extract, 10 kg of clove listed in the Japanese Pharmacopeia may be dried, finely divided, and then soaked for 24 hours in 50 v/v% ethanol (prepared according to the Japanese Standards of Cosmetic Ingredients, using absolute ethanol and purified water) and pressed for separation to obtain the extract.

Pruritus, rough skin and sensitive skin according to the invention are those conditions of skin induced by binding of SCF released from cells to the SCF receptors of mast cells. Pruritus refers in a general sense to itching of the skin or scalp, and there may be mentioned, for example, pruritus due to dryness, atopic dermatitis and senile xerosis. Rough skin refers in a general sense to a state of deterioration of the skin, and there may be mentioned rough skin resulting from pruritus-induced flaking or aggravated deterioration due to atopic dermatitis, and rough skin caused by dryness. Sensitive skin refers to skin in a highly irritable state, and indicates a state of skin which is hyperactive with respect to external non-specific physicochemical stimulation such as, for example, flaking, hyperthermia, perspiration, sunlight, clothing, accretion and the like, or to stimulation by psychological stress.

A "whitening effect" according to the invention refers to improvement in skin spots, freckles, darkened skin and the like which are caused by excessive production of melanin induced by binding of cell-released SCF with pigment cell SCF receptors due to sunburn, dryness and other causes.

The medications for pruritus, rough skin, sensitive skin and skin whitening of the invention will ordinarily be used by adding the aforementioned SCF production/release-inhibiting active ingredients to aqueous solvents such as water or ethanol. There are no particular restrictions on the contents of the SCF production/release-inhibiting ingredients, but they are preferably in the range of 0.00001 to 0.01 wt% and especially about 0.0001 to 0.005 wt% based on solid portion. When the medicaments of the present invention are to be prepared as bath powders, they will normally be diluted by about 100-1000 fold when used and, therefore, the contents are preferably prescribed at high concentrations for such purposes. Lower alcohols are preferred as the aqueous solvents mentioned above, and the lower alcohol contents may comprise 20-80 wt%, and preferably 40-60 wt% of the medicaments of the invention.

In addition to the essential ingredients described above, the medicaments for pruritus, rough skin, sensitive skin and/or skin whitening of the invention may also contain, as necessary and appropriate, ingredients commonly used for cosmetic and medical skin external preparations, such as other skin whiteners, moisteners, antioxidants, oil components, ultraviolet absorbers, surfactants, thickeners, alcohols, powdered ingredients, pigments, aqueous ingredients, water, various skin nutrients, and the like.

Also, depending on the purpose of the medicament, it may also contain, as appropriate, metal sequestering agents such as disodium edetate, trisodium edetate, sodium citrate, sodium polyphosphate, sodium metaphosphate or gluconic acid, drug agents such as caffeine, tannin, verapamil, tranexamic acid and its derivatives, licorice extract, glabridin, quince fruit hot water extract, various galenicals, tocopherol acetate or glycyrrhetinic acid and its derivatives or salts, other whiteners such as vitamin C, magnesium ascorbic acid phosphate, ascorbic acid glucoside, albutin or kojic acid, saccharides such as glucose, fructose, mannose, sucrose or trehalose, and vitamin A compounds such as retinoic acid, retinol, retinol acetate, retinol palmitate and the like.

The medicaments for pruritus, rough skin, sensitive skin and skin whitening of the invention may be in any form depending on the intended purpose, so long as they are external preparations of cosmetics, pharmaceuticals, quasi drugs or the like, and examples include forms conventionally used for skin external preparations such as, for example, cosmetic water, creams, emulsions, lotions, packs, bath additives, ointments, hair lotions, hair tonics, hair liquids, shampoos, rinses, hair-stimulating or hair-growth agents and the like.

### Examples

The present invention will now be explained in greater detail using the following examples.

### Experimental Example 1

### Assay of SCF released by keratinocytes with drying stimulation and screening for inhibiting agents

Commercially available human epidermal keratinocytes (neonatal; Cryo NHEK-Neo, Sanko Junyaku Co., Ltd.) were cultured using commercially available serum-free medium (Defined Keratinocyte-SFM, Gibco Industries, Inc.). The cells were plated in a 12-well microplate at 1 x 10⁵ cells/mL and cultured at about 37°C for about 72 hours to confluency.

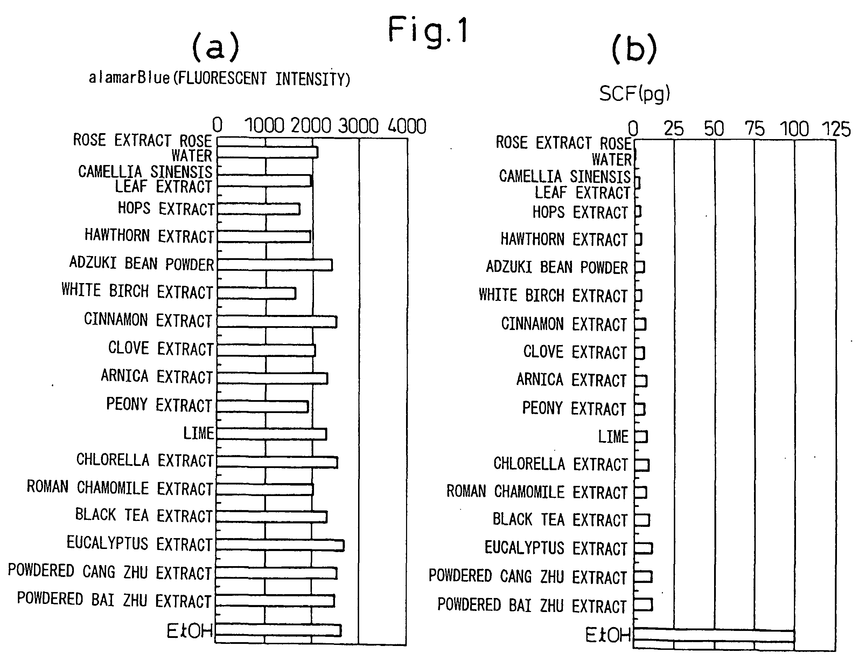

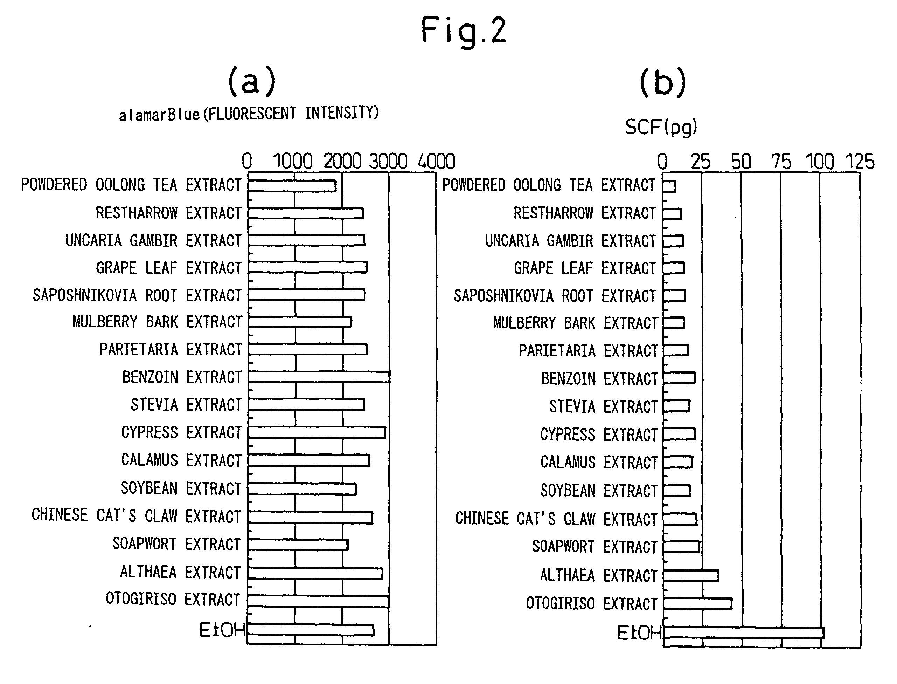

Each of the galenical extracts shown in Figs. 1 and 2, extracted with water or ethanol, was dissolved in 70% ethanol to 2% w/v.

Each test galenical extract was added to a final concentration of 0.005% w/v in the culture solution and incubated at 37°C for 24 hours. Cultures containing ethanol (EtOH) alone were also incubated as a control. In order to examine the cell activity, i.e. in order to determine the cytotoxic effects of the test galenical extracts, alamarBlue™ (Biosource International) was added at 10% during the final 2 hours and the fluorescent intensity (excitation: 560 nm, emission: 590 nm) of each supernatant was measured.

Drying stimulation was carried out by complete removal of the supernatant and holding in a CO₂ incubator for one hour.

Medium was then added and the supernatant was collected after 2 hours. The SCF level in the supernatant was quantitated using a commercially available ELISA assay kit (R&D Systems).

The SCF level-inhibiting effect of each test galenical extract was evaluated against the same test extract without drying stimulation.

The cytotoxicity of each test galenical extract was also evaluated using the amount of reduction of alamarBlue™ as the index.

The results are shown in Figs. 1 and 2. Figs. 1(a) and 2(a) show the amounts of reduction in fluorescent intensity of alamarBlue™ with addition of each galenical extract, and Figs. 1(b) and 2(b) show the values after subtracting the free SCF in the cell medium with addition of the galenical extract but without drying stimulation, from the amount of free SCF in the medium of the cells with addition of each galenical extract and with drying stimulation. As clearly seen by the results in Figs. 1 and 2, the galenical extracts significantly reduced SCF levels in the culture solutions compared to ethanol, and can therefore effectively inhibit production and/or release of SCF.

### Experimental Example 2

### Accelerated SCF expression on keratinocyte membranes by different stimulations

After plating 1.5 million keratinocytes on a 10 cm plate and culturing for 72 hours, the cells were stimulated by one of the following: (1) exchanging the medium with PBS(-), irradiating the culture with UVB at 20 mJ/cm² and then immediately exchanging the PBS(-) with medium, (2) adding forskolin, or (3) adding theophyllin. After culturing for 24 hours, the cells were collected and then dispersed in 200 µl of 50 mM phosphate buffer solution (pH 7.8) + protease inhibitor solution, and the cells were then disrupted with an ultrasonic disrupter for five 30-second disruption cycles at 4°C. After centrifugation at 10,000 g for 20 minutes at 4°C, the supernatant was further centrifuged at 100,000 g for 60 minutes at 4°C. The obtained pellet was dissolved in 100 µl of 25 mM phosphate buffer solution (pH 6.8) + 0.1% Triton-X100 solution and a membrane fraction protein extract was obtained. The protein content of the solution was measured by an established method, and then the SCF amount was assayed to obtain an SCF/protein value.

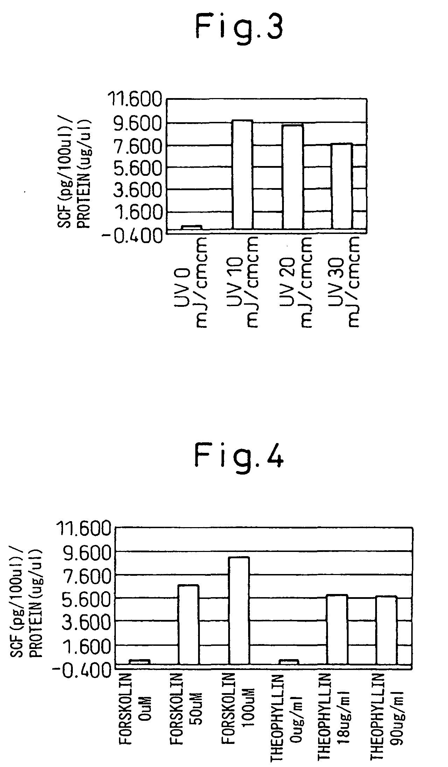

The results are shown in Figs. 3 and 4. As clearly shown by the results in these graphs, stimulation of the cells by ultraviolet stimulation or by chemical stimulation with forskolin or theophyllin accelerated expression of SCF in the cells or on the cell membranes.

### Experimental Example 3

### Assay of SCF expressed on keratinocyte membranes by ultraviolet ray stimulation and screening for inhibiting agents

After plating 1.5 million keratinocytes on a 10 cm plate and culturing for 72 hours, the medium was exchanged with PBS(-), the culture was irradiated with UVB at 20 mJ/cm² and the PBS(-) was immediately exchanged with medium. After adding the galenical to be screened and culturing for 24 hours, the cells were collected and then dispersed in 200 µl of 50 mM phosphate buffer solution (pH 7.8) + protease inhibitor solution, and the cells were disrupted with an ultrasonic disrupter for five 30-second disruption cycles each at 4°C. After centrifugation at 10,000 g for 20 minutes at 4°C, the supernatant was further centrifuged at 100,000 g for 60 minutes at 4°C. The obtained pellet was dissolved in 100 µl of 25 mM phosphate buffer solution (pH 6.8) + 0.1% Triton-X100 solution and a membrane fraction protein extract was obtained. The protein content of the solution was measured by an established method, and then the SCF amount was assayed to obtain an SCF/protein value. Inhibitors were defined as those galenicals among the tested galenical-added groups which produced a reduction of the value to 25% or lower compared to addition of the solvent alone.

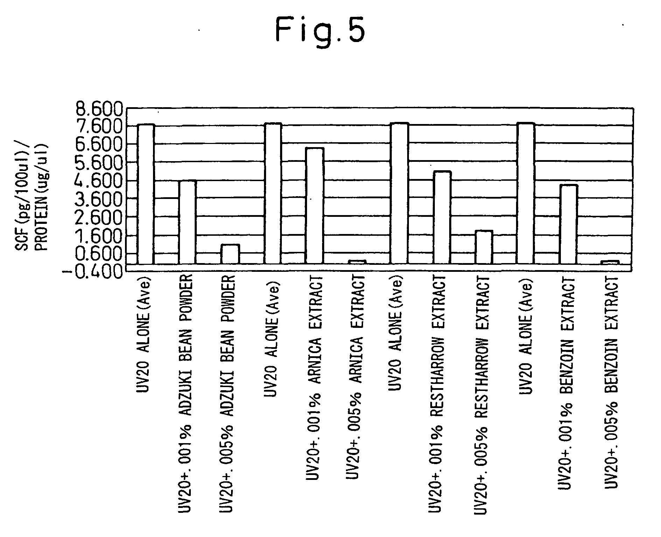

The results are shown in Fig. 5. As clearly shown by the results in this graph, the cells treated with the galenical extracts had significantly inhibited SCF expression compared to the cells treated only with ultraviolet irradiation.

Different formulations of galenical extract-containing medications according to the invention are listed below.

### Example 1

### Body cream

| | Content (pts. by wt.) |
|---|---|
| Methylpolysiloxane | 3 |
| Decamethylcyclopentasiloxane | 13 |
| Octamethylcyclotetrasiloxane | 12 |
| Polyoxyethylene-methylpolysiloxane copolymer | 1 |
| Ethanol | 2 |
| Isopropanol | 1 |
| Glycerin | 3 |
| Dipropylene glycol | 5 |
| Polyethylene glycol 6000 | 5 |
| Sodium metaphosphate | 0.05 |
| Potassium DL-α-tocopherol 2-L-ascorbic acid | |
| phosphoric acid diester | 0.1 |
| DL-α-tocopherol acetate | 0.1 |
| Caffeine | 0.1 |
| Fennel extract | 0.1 |
| Hamamelis extract | 0.1 |
| Ginseng extract | 0.1 |
| Powdered cang zhu extract | 0.005 |
| L-menthol | q.s. |
| Paraoxybenzoic acid ester | q.s. |
| Trisodium edetate | 0.05 |
| Dimorpholinopyridazine | 0.01 |
| Methylbis(trimethylsiloxy)silylisopentyl | |
| trimethoxycinnamate | 0.1 |
| Yellow iron oxide | q.s. |
| Cobalt titanate | q.s. |
| Dimethyldistearylammonium hectorite | 1.5 |
| Polyvinyl alcohol | 0.1 |
| Hydroxyethyl cellulose | 0.1 |
| Purified water | remainder |
| Trimethylsiloxycinnamic acid | 2 |
| Aromatic | q.s. |

### Example 2

### Emulsion

| | Content (pts. by wt.) |
|---|---|
| Methylpolysiloxane | 2 |
| Behenyl alcohol | 1 |
| Xylitol | 1 |
| Batyl alcohol | 0.5 |
| Glycerin | 5 |
| 1,3-Butylene glycol | 7 |
| Erythritol | 2 |
| Hydrogenated oil | 3 |
| Squalane | 6 |
| Pentaerythritol tetra(2-ethylhexanoate) | 2 |
| Polyoxyethylene glyceryl isostearate | 1 |
| Polyoxyethylene glycerin monostearate | 1 |
| Benzoin extract | 0.001 |
| Potassium hydroxide | q.s. |
| Sodium hexametaphosphate | 0.05 |
| Phenoxyethanol | q.s. |
| Carboxyvinyl polymer | 0.11 |
| Purified water | remainder |

### Example 3

### Lotion

| | Content (pts. by wt.) |
|---|---|
| Glycerin | 2 |
| 1,3-Butylene glycol | 4 |
| Erythritol | 1 |
| Polyoxyethylene methylglucoside | 1 |
| Polyoxyethylene hydrogenated castor oil | 0.5 |
| Citric acid | 0.02 |
| Sodium citrate | 0.08 |
| Phenoxyethanol | 0.25 |
| N-coconut oil fatty acid acyl L-arginine ethyl | |
| DL-pyrrolidonecarboxylate | 0.1 |
| Chinese cat's claw extract | 0.0001 |
| Purified water | remainder |

### Example 4

### Whitening lotion

| | Content (pts. by wt.) |
|---|---|
| Ethyl alcohol | 10 |
| Dipropylene glycol | 1 |
| Polyethylene glycol 1000 | 1 |
| Polyoxyethylene methylglucoside | 1 |
| Jojoba oil | 0.01 |
| Glyceryl tri(2-ethylhexanoate) | 0.1 |
| Polyoxyethylene hydrogenated castor oil | 0.2 |
| Polyglyceryl diisostearate | 0.15 |
| Sodium N-stearoyl-L-glutamate | 0.1 |
| Citric acid | 0.04 |
| Sodium citrate | 0.18 |
| Potassium hydroxide | 0.4 |
| Dipotassium glycyrrhizinate | 0.1 |
| Arginine hydrochloride | 0.1 |
| L-ascorbic acid 2-glucoside | 2 |
| Golden extract | 0.1 |
| Saxifrage extract | 0.1 |
| Paraben | 0.12 |
| Dead nettle extract | 0.1 |
| Dibutylhydroxytoluene | 0.01 |
| Trisodium edetate | 0.05 |
| 2-Ethylhexyl paramethoxycinnamate | 0.01 |
| Soapwort extract | 0.005 |
| Purified water | remainder |
| Mineral water | 3 |
| Aromatic | q.s. |

### Example 5

### Treatment Mask

| | Content (pts. by wt.) |
|---|---|
| Ethanol | 10 |
| 1,3-butylene glycol | 6 |
| Polyethylene glycol 4000 | 2 |
| Olive oil | 1 |
| Macadamia nuts | 1 |
| Phytosteryl hydroxystearate | 0.05 |
| Lactic acid | 0.05 |
| Sodium lactate solution (50%) | 0.2 |
| Disodium L-ascorbic acid sulfate ester | 0.1 |
| Potassium DL-α-tocopherol 2-L-ascorbic acid | |
| phosphoric acid diester | 0.1 |
| Vitamin E acetate | 0.1 |
| Fish collagen | 0.1 |
| Sodium chondroitin sulfate | 0.1 |
| Paraoxybenzoic acid ester | 0.1 |
| Carboxymethylcellulose sodium | 0.2 |
| Polyvinyl alcohol | 12.5 |
| Tranexamic acid | 1 |
| Powdered bai zhu extract | 0.001 |
| Purified water | remainder |
| Aromatic | q.s. |

### Example 6

### Whitening essence

| | Content (pts. by wt.) |
|---|---|
| Vaseline | 2 |
| Methylpolysiloxane | 2 |
| Ethyl alcohol | 5 |
| Behenyl alcohol | 0.5 |
| Batyl alcohol | 0.2 |
| Glycerin | 7 |
| 1,3-butylene glycol | 5 |
| Polyethylene glycol 20000 | 0.5 |
| Jojoba oil | 3 |
| Squalane | 2 |
| Cholesteryl hydroxystearate | 0.5 |
| Pentaerythritol tetra(2-ethylhexanoate) | 1 |
| Polyoxyethylene hydrogenated castor oil | 1 |
| Potassium hydroxide | 0.1 |
| Sodium pyrosulfite | 0.01 |
| Sodium hexametaphosphate | 0.05 |
| Stearyl glycyrrhizinate | 0.1 |
| Pantothenyl ethyl ether | 0.1 |
| Albutin | 7 |
| Tocopherol acetate | 0.1 |
| Sodium hyaluronate | 0.05 |
| Eucalyptus extract | 0.001 |
| Paraoxybenzoic acid ester | q.s. |
| Trisodium edetate | 0.05 |
| 4-t-Butyl-4'-methoxydibenzoylmethane | 0.1 |
| Glyceryl diparamethoxycinnamic | |
| mono-2-ethylhexanoate | 0.1 |
| Yellow iron oxide | q.s. |
| Xanthan gum | 0.1 |
| Carboxyvinyl polymer | 0.2 |
| Purified water | remainder |

### Example 7

### Bath additive for pruritus

| | Content (pts. by wt.) |
|---|---|
| Liquid paraffin | 35 |
| Polyethylene glycol | 20 |
| Squalane | 5 |
| Cetyl 2-ethylhexanoate | 5 |
| Polyoxyethylene hydrogenated castor oil | 5 |
| Citric acid | 0.1 |
| Sodium citrate | 0.2 |
| Glycine | 0.5 |
| Sodium hyaluronate | 0.3 |
| Phenoxyethanol | 0.5 |
| Parietaria extract | 1 |
| Purified water | remainder |
| Aromatic | q.s. |

### Industrial Applicability

According to the present invention it is possible to effectively screen for galenicals which inhibit production and release of SCF.

## Claims

1. A screening method for active ingredients which exhibit effects of ameliorating pruritus, rough skin or sensitive skin, or effects of skin whitening, by inhibiting production and/or release of stem cell factor (hereinafter, SCF), the method being **characterized by** comprising the steps of contacting SCF-expressing cells with test ingredients, assaying the amount of SCF produced and/or released by said cells, and selecting test ingredients which reduce the amount of production and/or release of SCF as said active ingredients, wherein said SCF-expressing cells are subjected to stimulation to promote SCF production and/or release.

2. The screening method according to claim 1, wherein said stimulation is drying stimulation, ultraviolet irradiation stimulation or chemical stimulation.

3. The screening method according to claim 1 or 2, wherein said SCF-expressing cells are contacted with said test ingredients, after which said cells are subjected to stimulation, and then the amount of SCF produced and/or released by said cells is assayed to select said active ingredients.

4. A skin external preparation which inhibits SCF production and/or release, which comprises one or more ingredients selected from the group consisting of rose extract rose water, camellia sinensis leaf extract, hops extract, hawthorn extract, adzuki bean powder, white birch extract, cinnamon extract, clove extract, arnica extract, peony extract, lime, chlorella extract, Roman chamomile extract, black tea extract, eucalyptus extract, powdered cang zhu extract, powdered bai zhu extract, powdered oolong tea extract, restharrow extract, uncaria gambir extract, grape leaf extract, saposhnikovia root extract, mulberry bark extract, parietaria extract, benzoin extract, stevia extract, cypress extract, calamus extract, soybean extract, Chinese cat's claw extract, soapwort extract, althaea extract, otogiriso (*Hypericum erectum*) extract and artemisia extract.

5. A skin external preparation for pruritus which comprises an ingredient which inhibits production and/or release of SCF as a pruritus-ameliorating active ingredient.

6. The skin external preparation for pruritus according to claim 5, wherein said pruritus-ameliorating active ingredient includes at least one selected from the group consisting of hops extract, hawthorn extract, adzuki bean powder, clove extract, Roman chamomile extract, black tea extract, eucalyptus extract, powdered cang zhu extract, powdered bai zhu extract, powdered oolong tea extract, restharrow extract, saposhnikovia root extract, parietaria extract, benzoin extract, stevia extract, calamus extract, Chinese cat's claw extract, soapwort extract, althaea extract and otogiriso (*Hypericum erectum*) extract.

7. A skin external preparation for rough skin prevention which comprises an ingredient which inhibits production and/or release of SCF as a rough skin-preventing active ingredient.

8. The skin external preparation for rough skin prevention according to claim 7, wherein said rough skin-preventing active ingredient includes at least one selected from the group consisting of hawthorn extract, powdered cang zhu extract, powdered bai zhu extract, restharrow extract, mulberry bark extract, parietaria extract, benzoin extract, stevia extract, cypress extract, calamus extract, Chinese cat's claw extract, soapwort extract and althaea extract.

9. A skin external preparation for sensitive skin which comprises an ingredient which inhibits production and/or release of SCF as a sensitive skin-ameliorating active ingredient.

10. The skin external preparation for sensitive skin according to claim 9, wherein said sensitive skin-ameliorating active ingredient includes at least one selected from the group consisting of rose extract rose water, camellia sinensis leaf extract, hops extract, hawthorn extract, adzuki bean powder, white birch extract, cinnamon extract, clove extract, chlorella extract, black tea extract, eucalyptus extract, powdered cang zhu extract, powdered bai zhu extract, powdered oolong tea extract, restharrow extract, uncaria gambir extract, grape leaf extract, saposhnikovia root extract, mulberry bark extract, parietaria extract, benzoin extract, stevia extract, cypress extract, calamus extract, soybean extract, Chinese cat's claw extract, soapwort extract, althaea extract, otogiriso (*Hypericum erectum*) extract and artemisia extract.

11. A skin external preparation for skin whitening which comprises an ingredient which inhibits production and/or release of SCF as a skin whitening active ingredient.

12. The skin external preparation for skin whitening according to claim 11, wherein said skin whitening active ingredient includes one or more selected from the group consisting of rose extract rose water, restharrow extract, uncaria gambir extract, parietaria extract, benzoin extract, soapwort extract, chlorella extract and eucalyptus extract.

13. A method for ameliorating pruritus, rough skin or sensitive skin or whitening skin, which comprises applying human or mammalian epidermis with an active ingredient which exhibits an effect of ameliorating pruritus, rough skin or sensitive skin, or an effect of skin whitening, by inhibiting production and/or release of SCF.

14. A medicament comprising an ingredient which inhibits SCF expressed inside or on the cell membranes of cornified cells in response to ultraviolet ray stimulation.

15. The medicament according to claim 14, wherein said ingredient is at least one selected from the group consisting of rose extract rose water, camellia sinensis leaf extract, hops extract, hawthorn extract, adzuki bean powder, white birch extract, cinnamon extract, clove extract, arnica extract, peony extract, lime, chlorella extract, Roman chamomile extract, black tea extract, eucalyptus extract, powdered cang zhu extract, powdered bai zhu extract, powdered oolong tea extract, restharrow extract, uncaria gambir extract, grape leaf extract, saposhnikovia root extract, mulberry bark extract, parietaria extract, benzoin extract, stevia extract, cypress extract, calamus extract, soybean extract, Chinese cat's claw extract, soapwort extract, althaea extract, otogiriso (*Hypericum erectum*) extract and artemisia extract.

16. The medicament according to claim 14, wherein the ingredient is adzuki bean powder, arnica extract, restharrow extract or benzoin extract.
